(19) Europäisches Patentamt
European Patent Office
Office européen des brevets

(11) **EP 4 524 974 A1**

(12) **EUROPEAN PATENT APPLICATION**
published in accordance with Art. 153(4) EPC

(43) Date of publication:
**19.03.2025 Bulletin 2025/12**

(21) Application number: **23803690.9**

(22) Date of filing: **28.03.2023**

(51) International Patent Classification (IPC):
***G16B 30/00*** *(2019.01)* ***G16B 20/00*** *(2019.01)*
***G16B 40/00*** *(2019.01)* ***G16B 50/00*** *(2019.01)*
***G16H 50/20*** *(2018.01)* ***G16H 50/30*** *(2018.01)*
***C12Q 1/6806*** *(2018.01)* ***C12Q 1/6827*** *(2018.01)*

(52) Cooperative Patent Classification (CPC):
**C12Q 1/6806; C12Q 1/6827; G16B 20/00; G16B 30/00; G16B 40/00; G16B 50/00; G16H 50/20; G16H 50/30**

(86) International application number:
**PCT/KR2023/004091**

(87) International publication number:
**WO 2023/219263 (16.11.2023 Gazette 2023/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.05.2022 KR 20220056483**
**08.06.2022 KR 20220069309**

(71) Applicant: **Genomecare Co., Ltd.**
**Suwon-si, Gyeonggi-do 16229 (KR)**

(72) Inventors:
- **KIM, Sunshin**
  **Yongin-si Gyeonggi-do 17003 (KR)**
- **PRASAD, Adhikari Krishna**
  **Hwaseong-si Gyeonggi-do 18261 (KR)**
- **JEONG, Jae Hwan**
  **Suwon-si Gyeonggi-do 16213 (KR)**
- **OH, Gyeong In**
  **Seoul 07051 (KR)**

(74) Representative: **HGF**
**HGF Limited**
**1 City Walk**
**Leeds LS11 9DX (GB)**

(54) **METHOD FOR DETECTING FETAL CHROMOSOMAL ANEUPLOIDIES ON BASIS OF FALSE-POSITIVE DATA AND FALSE-NEGATIVE DATA**

(57) Provided are a method for detecting fetal chromosomal aneuploidies on the basis of false-positive data and false-negative (normal) data and a computer-readable recording medium having recorded thereon a program applied to perform same. According to this invention, prenatal diagnosis for fetal chromosomal aneuploidies can be performed in a non-invasive manner with excellent sensitivity and specificity.

Figure 1d

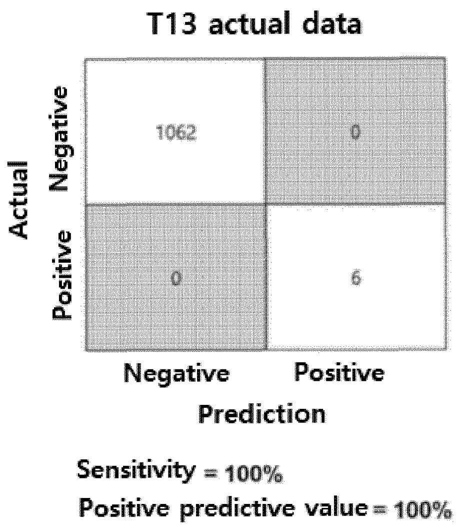


EP 4 524 974 A1

## Description

### Technical Field

There is provided a method for detecting chromosome aneuploidy of a fetus on the basis of synthetic positive data and synthetic negative data, i.e., synthetic chromosome aneuploidy data and synthetic normal data.

### Background Art

Prenatal diagnosis refers to diagnosing the presence or absence of a disease in the fetus before the fetus is born. Prenatal diagnosis is largely divided into invasive and non-invasive diagnostic methods. Invasive diagnostic methods include, for example, chorionic villus sampling, amniocentesis, and umbilical cord blood sampling. Invasive diagnostic methods have the potential to cause miscarriage, disease, or deformity by causing shock to the fetus during the test process, and thus non-invasive diagnostic methods that are performed prior to the invasive methods are being developed.

Recently, it has been demonstrated that non-invasive diagnosis of fetal chromosome aneuploidy is feasible by massive parallel sequencing of DNA molecules in the plasma of pregnant women. Fetal DNA can be detected in maternal plasma and serum from the 7th week of pregnancy, and the amount of fetal DNA in maternal blood increases with the period of pregnancy. When performing massive parallel sequencing of fetal DNA, the threshold for distinguishing a normal fetus from a chromosome aneuploidy fetus is unclear, resulting in low sensitivity and specificity for chromosome aneuploidy detection.

Therefore, there is a need to develop a method that can clearly distinguish a normal fetus from a chromosome aneuploidy fetus and increase the sensitivity and specificity of chromosome aneuploidy detection.

### Prior Art Document

### Patent Document

(Patent Document 1) KR 1739535 B

### Detailed Description of Invention

### Technical Problem

There is provided a method for detecting chromosome aneuploidy of a fetus.

There is provided a computer-readable medium for recording a program applied to perform the method.

### Solution to Problem

In one aspect, there is provided a method for detecting chromosome aneuploidy of a fetus, comprising:

obtaining sequence information (read) of a plurality of nucleic acid fragments obtained from a biological sample of a pregnant woman with a normal fetus;
mapping the obtained sequence information to a human reference genome to align the sequence information of the nucleic acid fragments to a chromosome;
calculating the read count (RC), fetal DNA fraction (fetal fraction: FF), and GC content of the sequence information of the nucleic acid fragments in the target chromosome in a normal fetus, based on the sequence information of the nucleic acid fragments aligned to the chromosome;
producing synthetic normal data from normal data based on the read count and fetal DNA fraction in the target chromosome in a normal fetus, wherein the normal data is the read count in the target chromosome in a normal fetus;
producing synthetic positive data from the synthetic normal data, wherein the synthetic positive data is the read count in the target chromosome in a fetus with chromosome aneuploidy;
establishing a chromosome aneuploidy discrimination model from the produced synthetic normal data and synthetic positive data; and
detecting chromosome aneuploidy of a test sample using the chromosome aneuploidy discrimination model.

The term "chromosome aneuploidy" refers to a condition in which the number of chromosomes per cell in a cell, individual, or lineage is not an integer multiple of the base number, and is one to several more or less than an integer multiple, that is, a condition involving a genome with an incomplete structure. In the case of diploidy, a case in which both two of a pair of homologous chromosomes are missing is referred to as nullisomy, and a case in which one of a pair of homologous chromosomes is missing and only the other is present is referred to as monosomy, and a case in which one extra chromosome is present in addition to one pair of homologous chromosomes is referred to as trisomy (T).

The chromosome aneuploidy may be trisomy 13, trisomy 18, trisomy 21, XO, XXX, XXY, XYY, or a combination thereof. Abnormalities of chromosome 13 (trisomy 13) are associated with Patau syndrome. Abnormalities of chromosome 18 (trisomy 18) are associated with Edwards syndrome. Abnormalities of chromosome 21 (trisomy 21) are associated with Down syndrome. Monosomy X (XO, i.e., the absence of one chromosome X) is associated with Turner syndrome. XXY is a condition in which human males have an extra chromosome X and is associated with Klinefelter syndrome.

The method comprises obtaining sequence information (read) of a plurality of nucleic acid fragments obtained from a biological sample of a pregnant woman with a normal fetus.

The pregnant woman may be a pregnant woman with a single fetus or twin fetuses.

The biological sample may be blood, plasma, serum, urine, saliva, mucosal secretions, sputum, feces, tears, or a combination thereof. The biological sample is, for example, peripheral blood plasma. The biological sample may include a nucleic acid of a fetus. The nucleic acid of a fetus may be cell-free DNA (cfDNA). The nucleic acid of a fetus may be isolated DNA.

The obtaining sequence information of a plurality of nucleic acid fragments obtained from a biological sample of a pregnant woman may comprise isolating nucleic acids from a biological sample.

The method of isolating nucleic acids from a biological sample may be performed by methods known to those of ordinary skill in the art. A length of the isolated nucleic acid fragment may be about 10 bp (base pair) to about 2000 bp, about 15 bp to about 1500 bp, about 20 bp to about 1000 bp, about 20 bp to about 500 bp, about 20 bp to about 200 bp, or about 20 bp to about 100 bp.

The obtaining sequence information of a plurality of nucleic acid fragments from a biological sample obtained from a pregnant woman may include performing massive parallel sequencing on the isolated nucleic acids.

The term "massive parallel sequencing" may be used interchangeably with next-generation sequencing (NGS) or second-generation sequencing. Massive parallel sequencing refers to a technique for simultaneously sequencing millions of fragments of nucleic acids. Massive parallel sequencing may be performed in parallel, for example, by 454 Platform (Roche), GS FLX Titanium, Illumina MiSeq, Illumina HiSeq, Illumina Genome Analyzer, Solexa platform, SOLiD System (Applied Biosystems), Ion Proton (Life Technologies), Complete Genomics, Helicos Biosciences Heliscope, single molecule real-time (SMRT™) technology from Pacific Biosciences, or a combination thereof.

The method may further comprise preparing a nucleic acid library in order to perform massive parallel sequencing.

The nucleic acid library may be prepared according to the method of massive parallel sequencing. The nucleic acid library may be constructed according to the manufacturer's instructions that provide massive parallel sequencing.

The sequence information of the obtained nucleic acid fragment may also be referred to as a read.

The method comprises mapping the obtained sequence information to a human reference genome to align the sequence information of the nucleic acid fragments to a chromosome.

The human reference genome may be hg18 or hg19. The sequence information that is mapped to only one genomic location in a human reference genome may be aligned as unique sequence information. Based on the aligned unique sequence number, the sequence information of the nucleic acid fragment may be aligned to the location of the chromosome. The location of the chromosome may be a contiguous range on a chromosome having a length of at least about 5 kb, about 10 kb, about 20 kb, about 50 kb, about 100 kb, about 1000 kb, or 2000 kb. The location of the chromosome may be a single chromosome.

After aligning the sequence information of the nucleic acid fragment to a chromosome, the method may further comprise checking the depth distribution of the sequence information of the nucleic acid fragments aligned to the chromosome for each section and excluding sections with low reliability of the sequence information from the analysis target. The section may be a section set in a unit of about 5 kb to about 50 kb. For example, the section may be a section set to about 10 kb to about 40 kb, about 15 kb to about 30 kb, or about 20 kb to about 25 kb. By setting the above section, filtering may be done using the GC content of the nucleotide sequence. In addition, by setting the above section, a group of the depth and GC content of the sequence information of the nucleic acid fragments aligned to the chromosome may be form, and statistical analysis may be performed.

The excluding sections with low reliability of the sequence information from the analysis target may comprise removing mismatch portions, removing the sequence information aligned to multiple sites, removing duplicated sequence information, or a combination thereof. In order to exclude sections with low reliability of the sequence information from the analysis target, quality filtering, trimming, perfect match, removal of sequences aligned to multiple locations, removal of PCR duplicated sequence information (PCR duplicated reads), or a combination thereof may be performed. The quality filtering is a process of extracting sequence information with high quality for the quality of each nucleotide sequence obtained during the sequencing process. The trimming is a process of removing parts of poor quality because the quality of the latter part of the nucleotide sequence is low due to the nature of the sequencing device. For example, nucleic acid fragments may be trimmed to a size of at least about 50 bp, greater than about 50 bp, or greater than about 100 bp. For example, the quality value of the nucleic acid fragment may be 20 or higher, 30 or higher, 40 or higher, or 50 or higher. The perfect match selects only nucleotide sequences that perfectly match when mapping to a human reference genome. Since sequences aligned to multiple locations are likely to be repetitive sequence regions, sequences aligned to multiple locations may be removed from the obtained sequence information. Removing the PCR duplicated sequence information means removing parts that have been amplified more due to errors during the sequencing process. In addition, for statistical analysis, a group with a certain degree of even deviation must be selected to obtain meaningful results. The part without depth is usually the N-region of the chromosome and may be excluded from the analysis target.

In the above method, based on the sequence information of the nucleic acid fragments aligned to the chromosome, the total number assigned to chromosome 1 may be denoted as C1RC (chromosome 1 read count), and the total number assigned to chromosome 2 may be denoted as C2RC (chromosome 2 read count). Therefore, the total number assigned to chromosome i may be denoted as CiRC (chromosome i read count). In addition, in the case of a sex chromosome, the total number assigned to chromosome X may be denoted as CXRC (chromosome X read count), and the total number assigned to chromosome Y may be denoted as CYRC (chromosome Y read count). The total number assigned to autosome 1 to autosome 22 is denoted as ATRC (autosomal total read count), and the total number assigned to all autosomes and sex chromosomes is denoted as TRC (total read count).

The method comprises calculating the read count (RC), fetal DNA fraction (fetal fraction: FF), and GC content of the sequence information of the nucleic acid fragments in the target chromosome in a normal fetus, based on the sequence information of the nucleic acid fragments aligned to the chromosome.

The target chromosome may be selected from the group consisting of chromosome 1 to chromosome 22, chromosome X, and chromosome Y. The target chromosome may be selected from the group consisting of chromosome 13, chromosome 18, and chromosome 21.

The term "read count (RC)" or "read number" is a value that counts the number of reads aligned to the location of one gene.

The term "fetal DNA fraction (fetal fraction: FF)" or "fraction of fetal nucleic acids" refers to the amount or ratio (%) of fetal nucleic acids among nucleic acids isolated from a biological sample of a pregnant woman. The fetal fraction may be a concentration, relative ratio, or absolute amount of fetal nucleic acids. The fetal nucleic acid may be a nucleic acid derived from fetal placenta trophoblasts.

The term "GC content" refers to the ratio (%) of guanine (G) and cytosine (C) among the bases that constitute DNA. The GC content may be calculated from the equation of GC content = (G+C)/(A+T+G+C).

The method comprises producing synthetic normal data from normal data based on the read count and fetal DNA fraction in the target chromosome in a normal fetus.

The normal data may be the read count in the target chromosome in a normal fetus. The normal data may also be referred to as negative data. The normal sample may be a normal male fetus sample or a normal female fetus sample.

The synthetic normal data may be the read count in the target chromosome obtained by randomly combining and dividing the normal data.

The producing synthetic normal data from normal data may be randomly selecting two or more samples from the normal samples, combining the selected fetus samples, and randomly dividing them again to produce synthetic normal data. In order to produce synthetic normal data from the normal data, two or more samples from the normal samples may be randomly selected, and the selected fetus samples may be combined to produce the entire sample. At this time, since each sample is a collection of reads, a desired amount of reads may be randomly selected from the entire sample to produce new synthetic normal data. The fetal DNA fraction and GC content of the synthetic normal data may be determined as follows. If the read count in sample A is represented as A, the read count in sample B is represented as B, the fetal DNA fraction in sample A is represented as Aff, and the fetal DNA fraction in sample B is represented as Bff, then the fetal DNA fraction in the entire sample may be $(A \times Aff + B \times Bff)/(A + B)$. The new synthetic normal data may also have the same fetal DNA fraction as that of the entire sample. The GC content may be calculated again from the new synthetic normal data.

The producing synthetic normal data from normal data may comprise distinguishing between normal male fetus samples and normal female fetus samples; and randomly selecting two or more samples from the normal male fetus samples, combining the selected male fetus samples, and randomly dividing them again to produce male fetus synthetic normal data, and/or randomly selecting two or more samples from the normal female fetus samples, combining the selected female fetus samples, and randomly dividing them again to produce female fetus synthetic normal data.

The method comprises producing synthetic positive data from the synthetic normal data.

The synthetic positive data is the read count in the target chromosome in a fetus with chromosome aneuploidy, and may be the synthetic read count obtained from the synthetic normal data.

The producing synthetic positive data may use CiRC (chromosome i read count) (i = 1 to 22) and FF in order to detect autosome aneuploidy. For example, in the case of chromosome 13, C13RC and FF are used.

The producing synthetic positive data from the synthetic normal data may be determined by the equation TiRC = CiRC + CiRC × FF/2 when the target chromosome is an autosome. In the above equation, TiRC is the read count in the target chromosome i in a chromosome aneuploidy fetus, CiRC is the read count in the target chromosome i in a normal fetus, and FF is the fetal DNA fraction.

The producing synthetic positive data from the synthetic normal data may be determined by the equation CXXXRC = CXXRC + CXXRC × FF/2 when the target chromosome is XXX chromosome. In the above equation, CXXXRC is the read count in the target chromosome XXX in a chromosome aneuploidy fetus, CXXRC is the read count in the target chromosome XX in a normal female fetus, and FF is the fetal DNA fraction.

The producing synthetic positive data from the synthetic normal data may be determined by the equation CXRC = CXYRC - CYRC + CyRC when the target chromosome is XO chromosome. In the above equation, CXRC is the read count in the target chromosome XO in a chromosome aneuploidy fetus, CXYRC is the read count in the target chromosome XY in a normal male fetus, CYRC is the read count assigned to chromosome Y in a normal male fetus, and CyRC is the read count incorrectly assigned to the target chromosome Y. The above CXRC, CXYRC, CYRC, and CyRC may be synthetic data.

The producing synthetic positive data from the synthetic normal data may be determined by the equation CXYYRC = CXYRC + CYRC - CyRC when the target chromosome is XYY chromosome. In the above equation, CXYYRC is the read count in the target chromosome XYY in a chromosome aneuploidy fetus, CXYRC is the read count in the target chromosome XY in a normal male fetus, CYRC is the read count assigned to chromosome Y in a normal male fetus, and CyRC is the read count incorrectly assigned to the target chromosome Y. The above CXYYRC, CXYRC, CYRC, and CyRC may be synthetic data.

The above CyRC may be calculated by a method comprising: calculating a residual from CyRC = $\beta_0 + \beta_1$ TRC + $\varepsilon$ (equation 1) and CyRC' = $\beta_0 + \beta_1$ TRC (equation 2); and calculating CyRC from the residual. In the above equation 1, TRC is the read count in the entire chromosome, $\beta_0$ is an intercept, $\beta_1$ is the coefficient between TRC and CyRC, and $\varepsilon$ is a residual. In the above equation 2, CyRC' is the predicted value of CyRC, TRC is the read count in the entire chromosome, $\beta_0$ is an intercept, and $\beta_1$ is the coefficient between TRC and CyRC'. The female fetus sample theoretically has no reads assigned to chromosome Y. However, some reads are incorrectly assigned to chromosome Y, and the read count tends to increase in proportion to the size of TRC. Therefore, the above equations 1 and 2 may be used to establish a model using a female fetus (XX) sample. The model established in this way may be approximately applied to a male fetus (XY) sample, which is different from a female fetus sample only in the sex chromosome. This is because the number of reads incorrectly assigned to chromosome Y in proportion to the size of TRC in a male fetus sample is similar to that in a female fetus sample. In other words, the TRC in a female fetus (XX) sample may be regarded as the TRC in a male fetus (XY) sample. The distribution of the residuals may be assumed to follow a normal distribution and may have a standard deviation that indicates the degree of spread of the normal distribution. By calculating CyRC' from equation 2, and using equation 1 to randomly generate residuals according to a normal distribution with the same standard deviation as the residual distribution of a female fetus sample described above and adding them to CyRC', the CyRC incorrectly assigned to chromosome Y in a male fetus sample may be approximately obtained.

The producing synthetic positive data from the synthetic normal data may be determined by the equation CXXYRC = CXXRC + CYRC when the target chromosome is XXY chromosome. In the above equation, CXXYRC is the read count in the target chromosome XXY in a chromosome aneuploidy fetus, CXXRC is the read count in the target chromosome XX in a normal female fetus, and CYRC is the read count assigned to chromosome Y.

The CYRC may be calculated by a method comprising calculating a residual from CYRC = $\beta_0 + \beta_1$ TRC + $\beta_2$ $FF_{snp}$ + $\varepsilon$ (equation 3) and CYRC' = $\beta_0$ + $B_1$ TRC + $\beta_2$ $FF_{snp}$ (equation 4); and calculating CYRC from the residual.

In the above equation 3, TRC is the read count in the entire chromosome, $FF_{snp}$ is FF based on the SNP, $\beta_0$ is an intercept, $\beta_1$ is the coefficient between TRC and CYRC, $\beta_2$ is the coefficient between $FF_{snp}$ and CYRC, and $\varepsilon$ is a residual. In the above equation 4, CYRC' is the predicted value of CYRC, TRC is the read count in the entire chromosome, $FF_{snp}$ is FF based on the SNP, $\beta_0$ is an intercept, $\beta_1$ is the coefficient between TRC and CYRC', and $\beta_2$ is the coefficient between $FF_{snp}$ and CYRC'. The method may comprise calculating CYRC from the calculated residual. The above equations 3 and 4 may be used to establish a model using a male fetus (XY) sample. The distribution of the residuals may be assumed to follow a normal distribution and may have a standard deviation indicating the degree of spread of the normal distribution. In addition, for samples with XXY chromosome aneuploidy, a regression model similar to that of male fetus samples and a normal distribution of residuals according to this model may be assumed. Since the read count assigned to chromosome Y (CYRC) is relatively small and negligible compared to the TRC, the TRC and FF of the female fetus (XX) sample may be considered to be the TRC and FF of the sample with XXY chromosome aneuploidy. By calculating CYRC' from equation 4, and using equation 3 to randomly generate residuals according to a normal distribution with the same standard deviation as the residual distribution of a male fetus sample described above and adding them to CYRC', the CYRC of a female fetus may be approximately obtained.

The method comprises establishing a chromosome aneuploidy discrimination model from the produced synthetic positive data and normal data.

The establishing a chromosome aneuploidy discrimination model from the produced synthetic positive data and normal data may be performed based on CiRC (chromosome i read count) (i = 1 to 22), ATRC (autosomal total read count), FF, and GC content when the target chromosome is an autosome.

The establishing a chromosome aneuploidy discrimination model from the produced synthetic positive data and normal data may be performed based on CXRC (chromosome X read count), CYRC (chromosome Y read count), TRC (total read count), FF, GC content, or a combination thereof when the target chromosome is a sex chromosome.

The chromosome aneuploidy discrimination model may be trained by applying a machine learning algorithm. The machine learning algorithm may be selected from the group consisting of LGBM (Light Gradient Boosting Machine), AdaBoost, Voting Classifier, Random Forest, logistic regression analysis (logistic algorithm), artificial neural network, and QDA (Quadratic Discriminant Analysis). The LGBM may be described in Guolin Ke et al., "LightGBM: A Highly Efficient Gradient Boosting Decision Tree". Proceedings of the 31st International Conference on Neural Information Processing Systems (NIPS 2017), pp. 3149-3157.

The method comprises detecting chromosome aneuploidy of a test sample using the chromosome aneuploidy discrimination model.

The detecting chromosome aneuploidy of a test sample using the chromosome aneuploidy discrimination model may be performed based on CiRC (i = 1 to 22), ATRC, FF, and GC content when the target chromosome is an autosome.

The detecting chromosome aneuploidy of a test sample using the chromosome aneuploidy discrimination model may be performed based on CXRC, CYRC, TRC, FF, GC content, or a combination thereof when the target chromosome is a sex chromosome.

In another aspect, there is provided a computer-readable medium for recording a program applied to perform the method according to one aspect. The computer-readable medium encompasses a system that comprises a computer-readable medium.

**Effects of Invention**

According to a method for detecting chromosome aneuploidy of a fetus on the basis of synthetic data according to an embodiment, and a computer-readable medium for recording a program applied to perform the method, non-invasive prenatal diagnosis of chromosome aneuploidy in a fetus can be performed with excellent sensitivity and specificity.

**Brief Description of Drawings**

Figure 1a shows the distribution of synthetic positive data and synthetic negative data according to three axes (ATRC, GC content, and C13RC) targeting chromosome 13 in three dimensions, Figure 1b shows the distribution of synthetic positive data and synthetic negative data according to GC content and C13RC in a plane, Figure 1c shows the results showing the sensitivity and positive predictive value of a test sample that was learned using 80% of the data (learning data) and verified using 20% of the data (test data) after randomly mixing data in a model using synthetic data, and Figure 1d shows the results obtained by further verifying the accuracy of the established model using a positive sample definitely diagnosed by amniocentesis.

Figure 2a shows the distribution of synthetic positive data and synthetic negative data according to three axes (ATRC, GC and C18RC) targeting chromosome 18 in three dimensions, Figure 2b shows the distribution of synthetic positive data and synthetic negative data according to GC content and C18RC in a plane, Figure 2c shows the results showing the accuracy of a test sample that was learned using 80% of the data (learning data) and verified using 20% of the data (test data) after randomly mixing data in a model using synthetic data, and Figure 2d shows the results obtained by further verifying the accuracy of the established model using a positive sample definitely diagnosed by amniocentesis.

Figure 3a shows the distribution of synthetic positive data and synthetic negative data according to three axes (ATRC, GC content, and C21RC) targeting chromosome 21 in three dimensions, Figure 3b shows the distribution of synthetic positive data and synthetic negative data according to GC content and C21RC in a plane, Figure 3c shows the results showing the accuracy of a test sample that was learned using 80% of the data (learning data) and verified using 20% of the data (test data) after randomly mixing data in a model using synthetic data, and Figure 3d shows the results obtained by further verifying the accuracy of the established model using a positive sample definitely diagnosed by amniocentesis.

Figure 4a shows the distribution of samples of a synthetic normal female fetus (XX) and synthetic positive data, a X monosomy (XO) and a triple X syndrome (XXX), according to two axes (GC content and CXRC) targeting the female fetus XX chromosome, Figure 4b shows the distribution of data of a synthetic normal male fetus (XY) and synthetic positive data, a Klinefelter syndrome (XXY) and a XYY syndrome (XYY), according to two axes (GC and CYRC) targeting the male fetus XY chromosome, Figure 4c shows the results showing the accuracy of a test sample that was learned using 80% of the data (learning data) and verified using 20% of the data (test data) after randomly mixing data in a model using synthetic positive data and synthetic negative data, and Figure 4d shows the results obtained by further verifying the accuracy of the established model using a positive sample definitely diagnosed by amniocentesis.

## Best Mode for Carrying out the Invention

Hereinafter, the present invention will be described in more detail through examples. However, these examples are intended to illustrate the present invention, and the scope of the present invention is not limited to these examples.

## Example 1. Non-invasive detection of fetal chromosome aneuploidy based on synthetic data production

### 1. Preparation of samples

Blood from a total of 15,999 pregnant women with a fetus was collected. Among these, amniocentesis for karyotyping of the fetus was performed on those subjects who tested positive.

All subjects underwent standard prenatal chromosome aneuploidy screening at a accredited clinical trial center. First trimester screening includes measurements of serum pregnancy-associated plasma protein A (PAPP-A), total or free beta subunit of human chorionic gonadotropin (hCG), and fetal nuchal translucency. Second trimester screening includes measurements of maternal serum alpha-fetoprotein (MSAFP), hCG, unconjugated estriol, and inhibin A.

As a result of karyotype analysis, 6 fetuses had aneuploidy on chromosome 13, 7 fetuses had aneuploidy on chromosome 18, 94 fetuses had aneuploidy on chromosome 21, 6 fetuses had an XO chromosome, 9 fetuses had an XXY chromosome, 2 fetuses had an XYY chromosome, and 13 fetuses had an XXX chromosome, indicating that chromosome aneuploidy was definitely diagnosed in the actual data.

### 2. Preparation of cell-free DNA and DNA library for DNA sequencing

Approximately 10 ml of peripheral blood was drawn from the subjects as described in 1. and collected in a BCT™ tube (Streck, Omaha, NE, USA). The collected blood samples were centrifuged at 1,200 $\times$ g at 4 °C for 15 minutes. Blood plasma was collected and centrifuged again at 16,000 g at 4 °C for 10 minutes. Cell-free DNA (cfDNA) was obtained from the centrifuged plasma using the MagListo™ cfDNA extraction kit (Bioneer, Korea).

The obtained cfDNA fragment was end-repaired using T4 DNA polymerase, Klenow DNA polymerase, and T4 polynucleotide kinase, and the cfDNA fragment was obtained again using the Agencourt AMPure XP.

A DNA library for the ion proton sequencing system was constructed from the prepared cfDNA according to the protocol provided by the manufacturer (ThermoFisher Scientific). Using the 540 chip, an average 0.3x sequencing coverage depth per nucleotide was calculated.

### 3. Massive parallel sequencing

The DNA library prepared as described in 2. was subjected to massive parallel sequencing using the Ion Torrent S5XL™ system (ThermoFisher Scientific).

Different raw reads were obtained using Ion Torrent Suite™ software (ThermoFisher Scientific). The filtered reads were aligned to the human genome reference sequence hg19 by Burrows-Wheeler transform (BWT). Sequence reads that were mapped to only one genomic location in hg19 were aligned as unique reads. Of the total reads, approximately $3.3 \times 10^6$ were unique reads, and the GC content of the total 15,999 samples ranged from approximately 39% to 45%.

Among the 15,999 samples described in 1., 348 male fetus samples and 354 female fetus samples were selected, which had the unique read count of 2 million or more, the GC content of 39.5% or more and less than 42%, and the fetal DNA fraction of 4% or more. The fetal DNA fraction was determined using SNP genotyping and imputation method (Korean Patent Registration No. KR 10-2031841) for the selected samples. At this time, the MAF filtering criterion value was set to 7%, and the correlation coefficient with the fetal DNA fraction based on chromosome Y was 98%.

In order to produce synthetic normal (negative) data, male fetuses and female fetuses were separated from each other, and two samples were randomly selected, combined, and randomly divided in half, but the two samples were selected so that no two samples overlapped. In other words, two samples were randomly selected from 348 male fetuses, combined, and then randomly divided in half again to produce 5,000 new male fetus synthetic normal data. At this time, when combining into one and then dividing in half again, the read count corresponding to 50% of the total read count was randomly selected. In the case of the female fetus, the same method was performed on 354 samples to produce 5,000 female fetus synthetic normal data.

## 4. Production and detection of synthetic positive data with chromosome aneuploidy from synthetic normal (negative) data

### A. Method of producing synthetic positive data with trisomy from synthetic normal autosomal data

A method of producing a trisomy from an autosome is to add the read count (RC) of autosome i plus the result of multiplying the read count (RC) of autosome i by 50% of the fetal DNA fraction (fetal fraction: FF). In other words, this can be expressed as an equation: TiRC = CiRC + CiRC $\times$ FF/2. In the above equation, TiRC is the read count in the target chromosome i in a chromosome aneuploidy fetus, CiRC is the read count in the target chromosome i in a normal fetus, and FF is the fetal DNA fraction.

A specific method of producing chromosome aneuploidy by targeting an autosome is explained using chromosomes 13, 18, and 21 as examples.

When any one of samples is selected and chromosome 13 is targeted, the DNA reads of chromosome 13 are mixed with maternal and fetal reads. At this time, it is important to know the ratio of the DNA reads in a fetus as accurately as possible. Therefore, in the case of a male fetus and a female fetus, the fetal DNA fraction (FF) using the SNP-based prediction method (imputation) is used.

In the case of a normal fetus, the number of DNA reads assigned to chromosome 13 is determined by deriving from a pair of maternal chromosomes and a pair of fetal chromosomes. Therefore, the chromosomal DNA read count in a normal fetus is determined by multiplying the total DNA read count assigned to chromosome 13 by the fetal DNA fraction (FF). On the other hand, because trisomy 13 has one additional fetal chromosome, an additional 50% must be added to the normal fetal DNA read count. As a result, if the DNA read count assigned to chromosome 13 in any sample is C13RC, chromosome 13 T13RC of the trisomy sample can be calculated by C13RC + C13RC $\times$ FF/2. As such, T18RC and T21RC can be obtained by applying this method to chromosomes 18 and 21.

### B. Detection of positive data with trisomy in autosome

#### (1) Detection of trisomy chromosome 13

When targeting chromosome 13, 10,000 synthetic positive data with trisomy were produced from a total of 10,000 synthetic normal data. After excluding outliers that were statistically outside the normal range in the synthetic data, 9,823 synthetic normal data and 9,646 synthetic positive data were selected.

The results of displaying the distribution of synthetic positive data and synthetic normal data according to three axes (ATRC, GC content, and C13RC) targeting chromosome 13 in three dimensions are shown in Figure 1a. In addition, the results of displaying the distribution of synthetic positive data and synthetic normal data according to GC content and C13RC in a plane are shown in Figure 1b. Figure 1a shows the distribution between the total read count of DNA reads assigned to autosomes (autosomal total read count: ATRC), and the total read count of DNA reads assigned to chromosome 13 (C13RC), and GC content for trisomy chromosome 13 (T13) and normal chromosome 13 (Normal). From these results, ATRC, C13RC, and GC content were confirmed to serve as parameters that can classify T13 and normal samples. In particular, Figure 1b shows that C13RC according to changes in GC content within the limited range of ATRC ($3.00 \times 10^6$ to $3.05 \times 10^6$) can be clearly distinguished between trisomy data and normal data.

After randomly mixing 9,823 synthetic normal data and 9,646 synthetic positive data using three parameters of ATRC, C13RC, and GC content, a model was established by learning with a logistic regression algorithm using 80% of the data (learning data). The sensitivity and positive predictive value of the test sample that was verified using the remaining 20% of the data (test data) were calculated, and the calculated results are shown in Figure 1c. As shown in Figure 1c, it was confirmed that the sensitivity and positive predictive value were each at least 99.99% in the learning and test data.

In order to confirm the accuracy of the established model, the results of additional verification using a positive sample definitely diagnosed by amniocentesis are shown in Figure 1d. As shown in Figure 1d, when this model using synthetic data was used, it was accurately predicted that the six actual positive samples (T13) confirmed by amniocentesis were positive data.

#### (2) Detection of trisomy chromosome 18

When targeting chromosome 18, 10,000 synthetic positive data with trisomy were produced from 10,000 synthetic normal data. Outliers were removed from the synthetic data, and 9,823 synthetic normal data and 9,529 synthetic positive data were selected. Data was selected within the ATRC range ($3.00 \times 10^6$ to $3.05 \times 10^6$). Some data that were statistically outside the normal range were removed depending on the characteristics of each target chromosome.

The results of displaying the distribution of synthetic positive data and synthetic normal data according to three axes (ATRC, GC and C18RC) targeting chromosome 18 in three dimensions are shown in Figure 2a. The results of displaying the distribution of synthetic positive data and synthetic normal data according to GC content and C18RC in a plane are shown in Figure 2b. A model was established by learning with a logistic regression algorithm using 80% of the data (learning data) after randomly mixing data using synthetic data. The results showing the sensitivity and positive predictive value of the test sample that was verified using the remaining 20% of the data (test data) are shown in Figure 2c. In order to confirm the accuracy of the established model, the results of additional verification using a positive sample definitely diagnosed by amniocentesis are shown in Figure 2d.

As shown in Figures 2a and 2b, ATRC, C18RC and GC content could distinguish trisomy chromosome 18 (T18) and normal chromosome (Normal). In addition, in Figure 2c, it was confirmed that the sensitivity and positive predictive value were each 100% in the learning and test data. As shown in Figure 2d, when the method using synthetic data was used, it was accurately predicted that the 7 actual positive samples (T18) confirmed by amniocentesis were positive data.

**(3) Detection of trisomy chromosome 21**

When targeting chromosome 21, 10,000 synthetic positive data with trisomy were produced from 10,000 normal synthetic data. Outliers were removed from the synthetic data, and 9,823 synthetic normal data and 9,594 synthetic positive data were selected. Data was selected within the ATRC range ($3.00 \times 10^6$ to $3.05 \times 10^6$). Some data that were statistically outside the normal range were removed depending on the characteristics of each target chromosome.

The results of displaying the distribution of synthetic positive data and synthetic normal data according to three axes (ATRC, GC and C21RC) targeting chromosome 21 in three dimensions are shown in Figure 3a. The results of displaying the distribution of synthetic positive data and synthetic normal data according to GC content and C21RC in a plane are shown in Figure 3b. A model was established by learning with a logistic regression algorithm using 80% of the data (learning data) after randomly mixing data using synthetic data. The results showing the sensitivity and positive predictive value of the test sample that was verified using the remaining 20% of the data (test data) are shown in Figure 3c. In order to confirm the accuracy of the established model, the results of additional verification using a positive sample definitely diagnosed by amniocentesis are shown in Figure 3d.

As shown in Figures 3a and 3b, ATRC, C21RC and GC content could distinguish trisomy chromosome 21 (T21) and normal chromosome (Normal). In addition, in Figure 3c, it was confirmed that the sensitivity and positive predictive value were 99.8% or more in the learning and test data. As shown in Figure 3d, when the method using synthetic data was used, it was accurately predicted that the 94 actual positive samples (T21) confirmed by amniocentesis were positive data.

**C. Method of producing synthetic positive data with sex chromosome aneuploidy from synthetic normal sex chromosome data**

In order to produce data with a sex chromosome aneuploidy in a fetus, male fetus (XY) data, female fetus (XX) data, and fetal DNA fraction (FF) were used.

For FF, FF based on the chromosome Y was used for male fetus samples, and FF based on machine learning was used for female fetus samples.

**(1) Detection of XO sex chromosome aneuploidy**

A method of producing synthetic positive data representing XO sex chromosome aneuploidy is to remove the read count assigned to chromosome Y (chromosome Y read count: CYRC) in male fetus samples from the read count (chromosome XY read count: CXYRC) in male fetus samples. However, there is a certain amount of reads incorrectly assigned to chromosome Y.

$$CXRC = CXYRC - CYRC + CyRC$$

In the above equation, CXRC is the read count in the target chromosome XO in a chromosome aneuploidy fetus, CXYRC is the read count in the target chromosome XY in a normal male fetus, CYRC is the read count in the target chromosome Y, and CyRC is the read count incorrectly assigned to the target chromosome Y.

The read count incorrectly assigned to chromosome Y (CyRC) is expressed as a regression equation proportional to the total read count (total size of read count).

That is, it is expressed as the regression equation of equation 1.

$$CyRC = \beta_0 + \beta_1 \, TRC + \varepsilon \text{ (equation 1)}$$

In the above equation 1, TRC is the read count in the entire chromosome, $\beta_0$ is an intercept, $\beta_1$ is the coefficient

between TRC and CyRC, and $\varepsilon$ is a residual.

The above equation 1 uses TRC as an independent variable. The predicted value of the dependent variable can be expressed as the following equation 2.

$$CyRC' = \beta_0 + \beta_1 \, TRC \text{ (equation 2)}$$

In the above equation 2, CyRC' is the predicted value of CyRC, TRC is the read count in the entire chromosome, $\beta_0$ is an intercept, and $\beta_1$ is the coefficient between TRC and CyRC'.

The equation is calculated using 8,737 female fetus samples as follows.

$$CyRC' = 3.8 \times 10^{-5} \, TRC + 8.7$$

From the above equations 1 and 2, the residual was calculated. The distribution of the residuals may be assumed to follow a normal distribution and may have a standard deviation indicating the degree of spread of the normal distribution. The standard deviation of the residuals was calculated as 14 from the normal distribution. A new residual was calculated by normal distribution according to this standard deviation. The total read count (TRC) of any male fetus and CyRC' are calculated from the above equation 2, and the new residual calculated above is added to calculate the new CyRC. As a result, the read count assigned to chromosome Y (chromosome Y read count: CYRC) can be removed from the CXYRC of any male fetus samples, and the newly obtained CyRC can be added to obtain CXRC.

**(2) Detection of XXX sex chromosome aneuploidy**

The method of producing the read count of a fetus with XXX sex chromosome aneuploidy is to add the read count in any female fetus samples (CXXRC) plus the read count (CXXRC) multiplied by 50% of FF. In other words, CXXXRC can be calculated by CXXXRC = CXXRC + CXXRC $\times$ FF/2. In the above equation, CXXXRC is the read count in the target chromosome XXX in a chromosome aneuploidy fetus, CXXRC is the read count in the target chromosome XX in a normal female fetus, and FF is the fetal DNA fraction.

**(3) Detection of XYY sex chromosome aneuploidy**

The method of producing the read count of a fetus with XYY sex chromosome aneuploidy is to add the read count in any male fetus samples (chromosome XY read count: CXYRC) plus the read count assigned to chromosome Y in the male fetus samples (chromosome Y read count: CYRC). In other words, when producing the read count of a fetus with XYY chromosome aneuploidy (chromosome XYY read count: CXYYRC), since the read count of CyRC is already included in CXYRC, adding CYRC would result in a duplicate, so CyRC is subtracted. In other words, it can be calculated by the following equation.

$$CXYYRC = CXYRC + CYRC - CyRC$$

In the above equation, CXYYRC is the read count in the target chromosome XYY in a chromosome aneuploidy fetus, CXYRC is the read count in the target chromosome XY in a normal male fetus, CYRC is the read count assigned to chromosome Y in a normal male fetus, and CyRC is the read count incorrectly assigned to the target chromosome Y.

Here, CyRC was calculated using the mathematical equation of equations 1 and 2 and the standard deviation of the residuals of 14.

**(4) Detection of XXY sex chromosome aneuploidy**

The method of producing the read count of a fetus with XXY sex chromosome aneuploidy (chromosome XXY read count: CXXYRC) can be calculated as CXXYRC = CXXRC + CYRC. In the above equation, CXYYRC is the read count in the target chromosome XYY in a chromosome aneuploidy fetus, CXYRC is the read count in the target chromosome XY in a normal female fetus, CYRC is the read count assigned to chromosome Y in a normal male fetus, and CyRC is the read count incorrectly assigned to the target chromosome Y.

First, CYRC is expressed as the regression equation of equation 3 using FF based on the chromosome Y.

$$CYRC = \beta_0 + \beta_1 \, TRC + \beta_2 \, FF_{snp} + \varepsilon \text{ (equation 3)}$$

In the above equation 3, TRC is the read count in the entire chromosome, $FF_{snp}$ is FF based on the SNP, $\beta_0$ is an intercept, $\beta_1$ is the coefficient between TRC and CYRC, $\beta_2$ is the coefficient between $FF_{snp}$ and CYRC, and $\varepsilon$ is a residual.

The above equation 3 uses TRC and FF based on the chromosome Y as an independent variable. The predicted value of the dependent variable can be expressed as the following equation 4.

$$CYRC' = \beta_0 + \beta_1\ TRC + \beta_2\ FF_{snp}\ \text{(equation 4)}$$

In the above equation 4, CYRC' is the predicted value of CYRC, TRC is the read count in the entire chromosome, $FF_{snp}$ is FF based on the SNP, $\beta_0$ is an intercept, $\beta_1$ is the coefficient between TRC and CYRC', and $\beta_2$ is the coefficient between $FF_{snp}$ and CYRC'.

The equation is calculated using 9,406 male fetus samples as follows.

$$CYRC' = 0.00018\ TRC + 72.5\ FF_{snp} - 576$$

From the above equations 3 and 4, the standard deviation of the residuals was calculated as 92, and a new residual was calculated by normal distribution according to this standard deviation. CYRC' are calculated from the above equation 4 using the total read count (TRC) in any female fetuses and FF based on the SNP, and the new residual obtained previously was added to calculate the new CYRC. As a result, CXXYRC can be obtained by adding the newly obtained CYRC to the CXXRC in any female fetus samples.

**D. Detection of positive data with sex chromosome aneuploidy on sex chromosomes**

A graph representing the distribution of samples of a synthetic normal female fetus (XX) and synthetic positive data, a X monosomy (XO) and a triple X syndrome (XXX) according to two axes (GC and CXRC) targeting the female fetus XX chromosome within the range ($3.00 \times 10^6$ to $3.05 \times 10^6$) of the total read count (TRC) in two dimensions in a plane is shown in Figure 4a. As shown in Figure 4a, there was a difference between the distributions of CXXRC, CXORC, and CXXXRC in the read count assigned to the chromosome X (CXRC) according to GC content in the target female fetus X chromosome (XX).

A graph representing the distribution of data of a synthetic normal male fetus (XY) and synthetic positive data, a Klinefelter syndrome (XXY) and a XYY syndrome (XYY) according to two axes (GC content and CYRC) targeting the male fetus XY chromosome within the range ($3.00 \times 10^6$ to $3.05 \times 10^6$) of the TRC in a plane is shown in Figure 4b. As shown in Figure 4b, there was a difference between the distributions of CXYRC, CXXYRC, and CXYYRC in the read count assigned to the chromosome Y (CYRC) according to GC content in the target male fetus chromosome Y (XY).

It was confirmed that due to these differences in distribution, CXRC, CYRC, and GC content can be used as parameters to classify a normal fetus sample such as XX and XY and a sex chromosome aneuploidy sample with XO, XXX, XXY, or XYY.

10,000 synthetic normal male fetuses and 9,994 synthetic normal female fetuses were used to produce 10,000 XYY, 10,000 XO, 9,957 XXX, and 9,967 XXY synthetic positive data, respectively. In addition, 10,000 synthetic normal male fetuses and 9,930 synthetic normal female fetuses were additionally produced and used without overlapping with the previously mentioned ones. Here, the synthetic data were randomly mixed, and 80% was used to establish a model by learning, and the results showing the accuracy of a test sample that was verified using the remaining 20% of the data are shown in Figure 4c. As shown in Figure 4c, it was confirmed that the learning and test data showed an accuracy of 99.8% or more in sensitivity and positive predictive value, respectively.

The results obtained by further verifying the accuracy of the established model using a positive sample definitely diagnosed by amniocentesis are shown in Figure 4d. As shown in Figure 4d, the actual chromosome aneuploidy (positive) samples of 6 XO, 9 XXY, 2 XYY, and 13 XXX confirmed by amniocentesis were predicted with 100% accuracy.

## Claims

**1.** A method for detecting chromosome aneuploidy of a fetus, comprising:

obtaining sequence information (read) of a plurality of nucleic acid fragments obtained from a biological sample of a pregnant woman with a normal fetus;
mapping the obtained sequence information to a human reference genome to align the sequence information of the nucleic acid fragments to a chromosome;
calculating the read count (RC), fetal DNA fraction (fetal fraction: FF), and GC content of the sequence information of the nucleic acid fragments in the target chromosome in a normal fetus, based on the sequence information of the nucleic acid fragments aligned to the chromosome;
producing synthetic normal data from normal data based on the read count and fetal DNA fraction in the target chromosome in a normal fetus, wherein the normal data is the read count in the target chromosome in a normal fetus;
producing synthetic positive data from the synthetic normal data, wherein the synthetic positive data is the read count in the target chromosome in a fetus with chromosome aneuploidy;
establishing a chromosome aneuploidy discrimination model from the produced synthetic normal data and synthetic positive data; and
detecting chromosome aneuploidy of a test sample using the chromosome aneuploidy discrimination model.

**2.** The method according to claim 1, wherein the target chromosome is selected from the group consisting of chromosome 1 to chromosome 22, chromosome X, and chromosome Y.

**3.** The method according to claim 2, wherein the target chromosome is selected from the group consisting of chromosome 13, chromosome 18, and chromosome 21.

**4.** The method according to claim 1, wherein producing synthetic normal data from normal data is randomly selecting two or more samples from the normal samples, combining the selected fetus samples, and randomly dividing them again to produce synthetic normal data.

**5.** The method according to claim 1, wherein producing synthetic normal data from normal data comprises

distinguishing between normal male fetus samples and normal female fetus samples; and
randomly selecting two or more samples from the normal male fetus samples, combining the selected male fetus samples, and randomly dividing them again to produce male fetus synthetic normal data, or
randomly selecting two or more samples from the normal female fetus samples, combining the selected female fetus samples, and randomly dividing them again to produce female fetus synthetic normal data.

**6.** The method according to claim 1, wherein producing synthetic positive data from the synthetic normal data is determined by the following equation when the target chromosome is an autosome:

$$\mathrm{TiRC} = \mathrm{CiRC} + \mathrm{CiRC} \times \mathrm{FF}/2,$$

in the above equation,

TiRC is the read count in the target chromosome i in a chromosome aneuploidy fetus,
CiRC is the read count in the target chromosome i in a normal fetus, and
FF is the fetal DNA fraction.

**7.** The method according to claim 1, wherein producing synthetic positive data from the synthetic normal data is determined by the following equation when the target chromosome is XXX chromosome:

$$\mathrm{CXXXRC} = \mathrm{CXXRC} + \mathrm{CXXRC} \times \mathrm{FF}/2,$$

in the above equation,

CXXXRC is the read count in the target chromosome XXX in a chromosome aneuploidy fetus,
CXXRC is the read count in the target chromosome XX in a normal female fetus, and
FF is the fetal DNA fraction.

**8.** The method according to claim 1, wherein producing synthetic positive data from the synthetic normal data is determined by the following equation when the target chromosome is XO chromosome:

$$\mathrm{CXRC} = \mathrm{CXYRC} - \mathrm{CYRC} + \mathrm{CyRC},$$

in the above equation,

CXRC is the read count in the target chromosome XO in a chromosome aneuploidy fetus,
CXYRC is the read count in the target chromosome XY in a normal male fetus,
CYRC is the read count in the target chromosome Y, and
CyRC is the read count incorrectly assigned to the target chromosome Y.

**9.** The method according to claim 1, wherein producing synthetic positive data from the synthetic normal data is determined by the following equation when the target chromosome is XYY chromosome:

$$CXYYRC = CXYRC + CYRC - CyRC,$$

in the above equation,

CXYYRC is the read count in the target chromosome XYY in a chromosome aneuploidy fetus,
CXYRC is the read count in the target chromosome XY in a normal male fetus,
CYRC is the read count assigned to chromosome Y in a normal male fetus, and
CyRC is the read count incorrectly assigned to the target chromosome Y.

**10.** The method according to claim 8 or 9, wherein the CyRC is calculated by a method comprising:

calculating a residual from the following equations 1 and 2:

$$CyRC = \beta_0 + \beta_1 \ TRC + \varepsilon \ (equation\ 1),$$

in the above equation 1, TRC is the read count in the entire chromosome, $\beta_0$ is an intercept, $\beta_1$ is the coefficient between TRC and CyRC, and $\varepsilon$ is a residual,

$$CyRC' = \beta_0 + \beta_1 \ TRC \ (equation\ 2),$$

in the above equation 2, CyRC' is the predicted value of CyRC, TRC is the read count in the entire chromosome, $\beta_0$ is an intercept, and $\beta_1$ is the coefficient between TRC and CyRC'; and
calculating CyRC from the residual.

**11.** The method according to claim 1, wherein producing synthetic positive data from the synthetic normal data is determined by the following equation when the target chromosome is XXY chromosome:

$$CXXYRC = CXXRC + CYRC,$$

in the above equation,

CXXYRC is the read count in the target chromosome XXY in a chromosome aneuploidy fetus,
CXXRC is the read count in the target chromosome XX in a normal female fetus, and
CYRC is the read count assigned to chromosome Y.

**12.** The method according to claim 11, wherein the CYRC is calculated by a method comprising:

calculating a residual from the following equations 3 and 4:

$$CYRC = \beta_0 + \beta_1 \ TRC + \beta_2 \ FF_{snp} + \varepsilon \ (equation\ 3),$$

in the above equation 3, TRC is the read count in the entire chromosome, $FF_{snp}$ is FF based on the SNP, $\beta_0$ is an intercept, $\beta_1$ is the coefficient between TRC and CYRC, $\beta_2$ is the coefficient between $FF_{snp}$ and CYRC, and $\varepsilon$ is a residual,

$$CYRC' = \beta_0 + \beta_1 \, TRC + \beta_2 \, FF_{snp} \text{ (equation 4)},$$

in the above equation 4, CYRC' is the predicted value of CYRC, TRC is the read count in the entire chromosome, $FF_{snp}$ is FF based on the SNP, $\beta_0$ is an intercept, $\beta_1$ is the coefficient between TRC and CYRC', and $\beta_2$ is the coefficient between $FF_{snp}$ and CYRC'; and
calculating CYRC from the residual.

**13.** The method according to claim 1, wherein establishing a chromosome aneuploidy discrimination model from the produced synthetic normal data and synthetic positive data is performed based on CiRC (chromosome i read count) (i = 1 to 22), ATRC (autosomal total read count), FF, GC content, or a combination thereof when the target chromosome is an autosome.

**14.** The method according to claim 1, wherein detecting chromosome aneuploidy of a test sample using the chromosome aneuploidy discrimination model is performed based on CiRC (i = 1 to 22), ATRC, FF, and GC content, or a combination thereof when the target chromosome is an autosome.

**15.** The method according to claim 1, wherein establishing a chromosome aneuploidy discrimination model from the produced synthetic normal data and synthetic positive data is performed based on CXRC (chromosome X read count), CYRC (chromosome Y read count), TRC (total read count), FF, GC content, or a combination thereof when the target chromosome is a sex chromosome.

**16.** The method according to claim 1, wherein detecting chromosome aneuploidy of a test sample using the chromosome aneuploidy discrimination model is performed based on CXRC, CYRC, TRC, FF, and GC content, or a combination thereof when the target chromosome is a sex chromosome.

**17.** The method according to claim 1, wherein the chromosome aneuploidy discrimination model is trained by applying a machine learning algorithm.

**18.** The method according to claim 17, wherein the machine learning algorithm is selected from the group consisting of LGBM (Light Gradient Boosting Machine), AdaBoost, Voting Classifier, Random Forest, logistic regression analysis (logistic algorithm), artificial neural network, and QDA (Quadratic Discriminant Analysis).

**19.** A computer-readable medium for recording a program applied to perform the method according to any one of claims 1 to 18.

Figure 1a

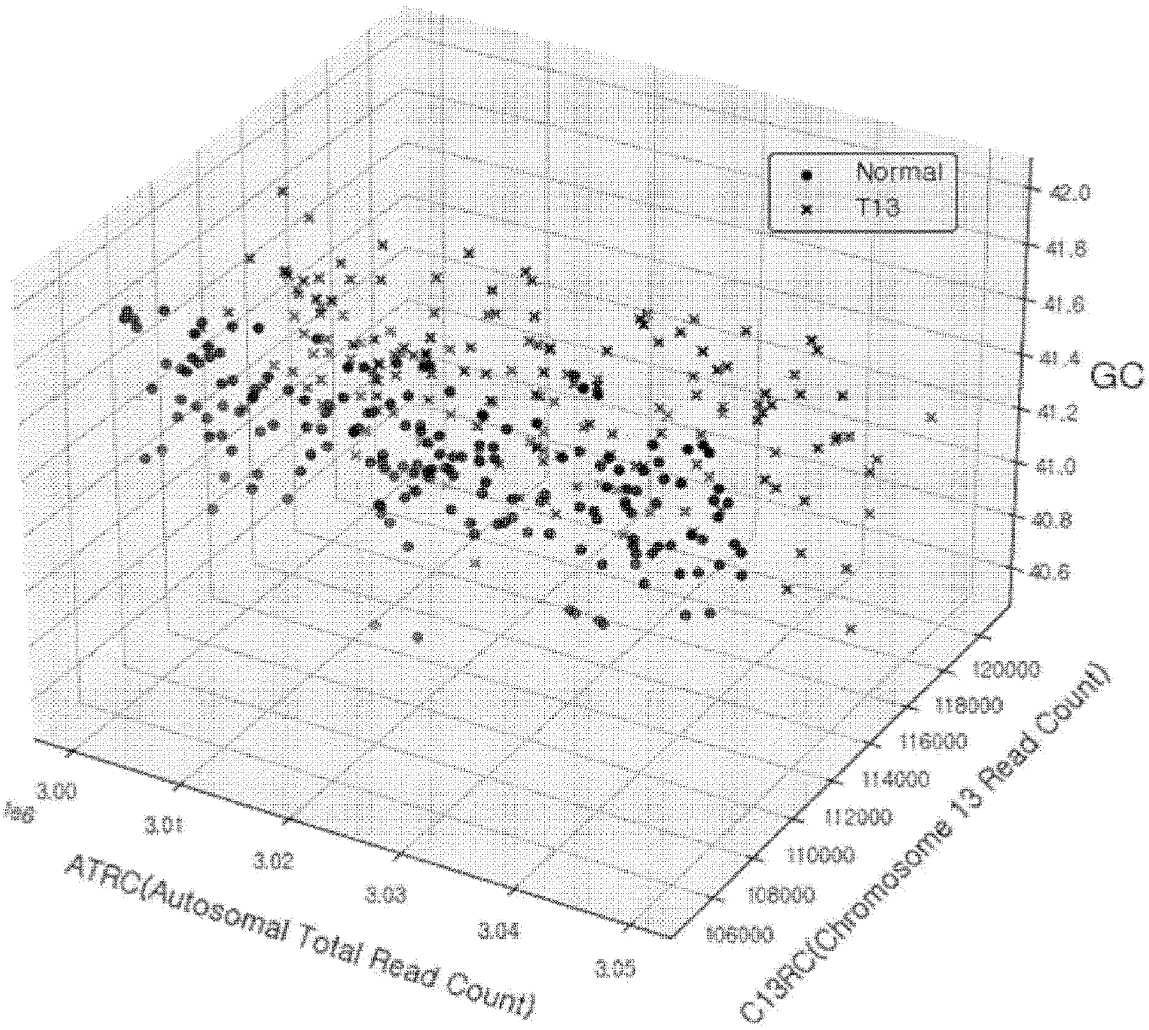
Normal
T13
GC
42.0
41.8
41.6
41.4
41.2
41.0
40.8
40.6
1e6
3.00
3.01
3.02
3.03
3.04
3.05
ATRC(Autosomal Total Read Count)
120000
118000
116000
114000
112000
110000
108000
106000
C13RC(Chromosome 13 Read Count)

Figure 1b

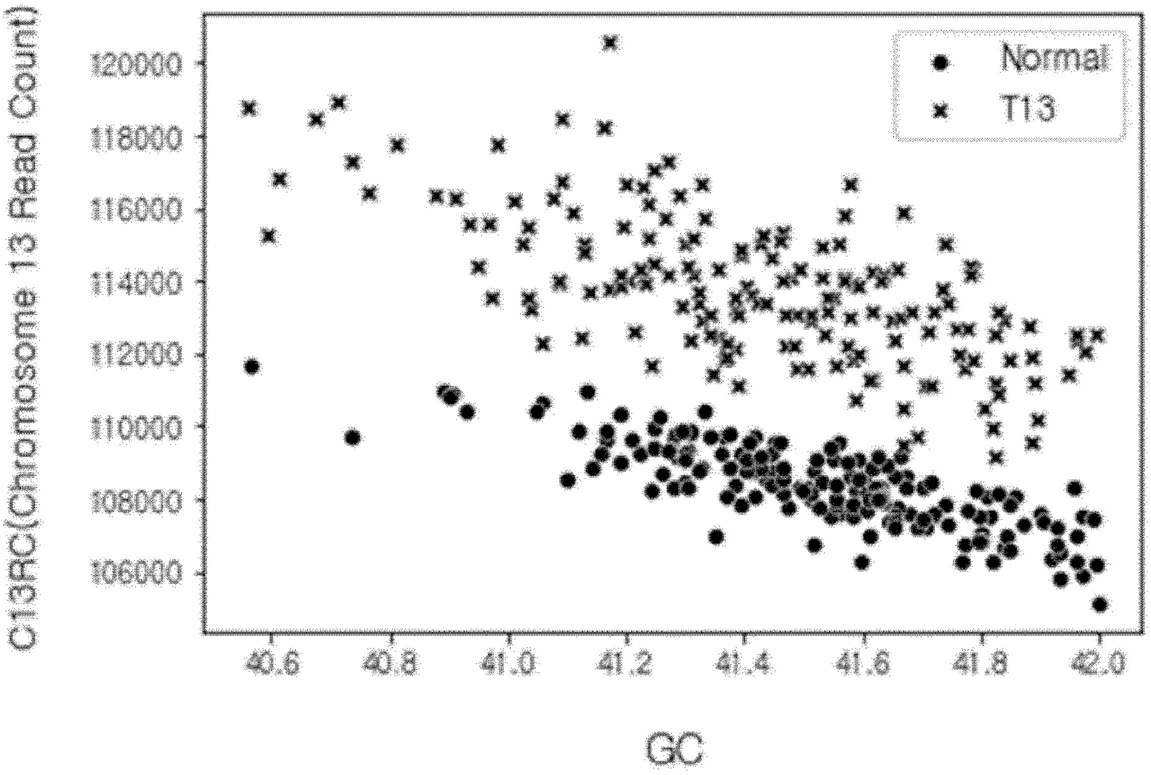
C13RC(Chromosome 13 Read Count)
120000
118000
116000
114000
112000
110000
108000
106000
40.6
40.8
41.0
41.2
41.4
41.6
41.8
42.0
GC
Normal
T13

Figure 1c

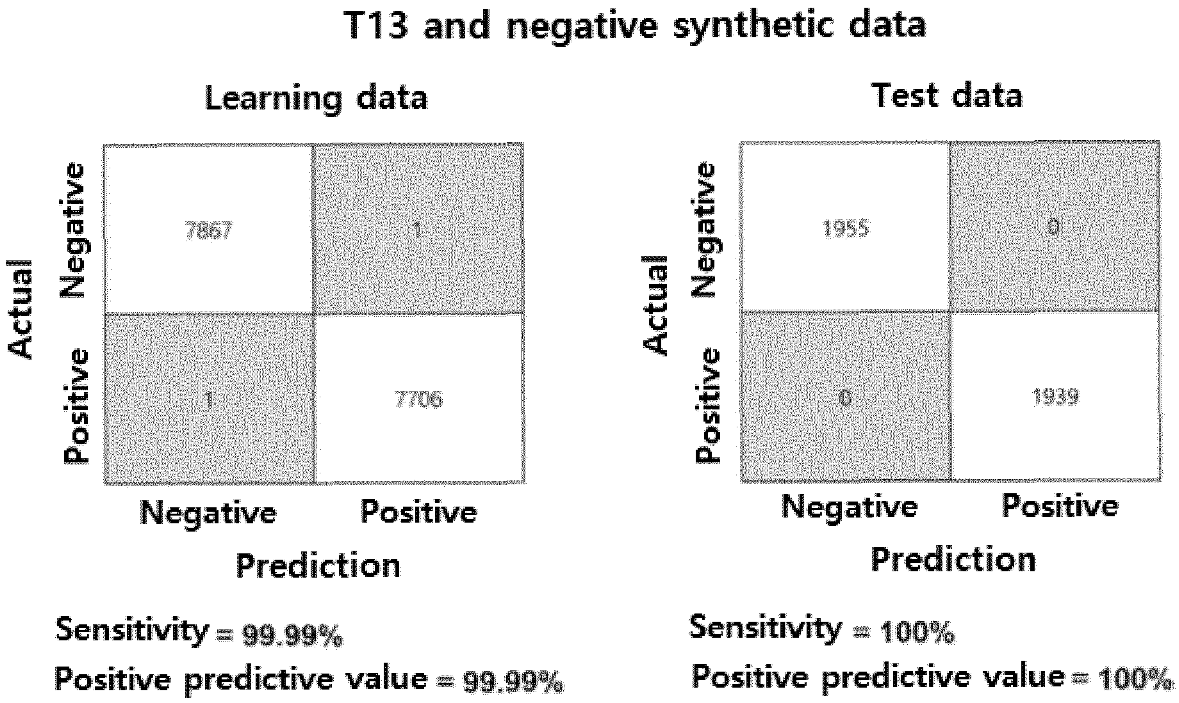


Figure 1d

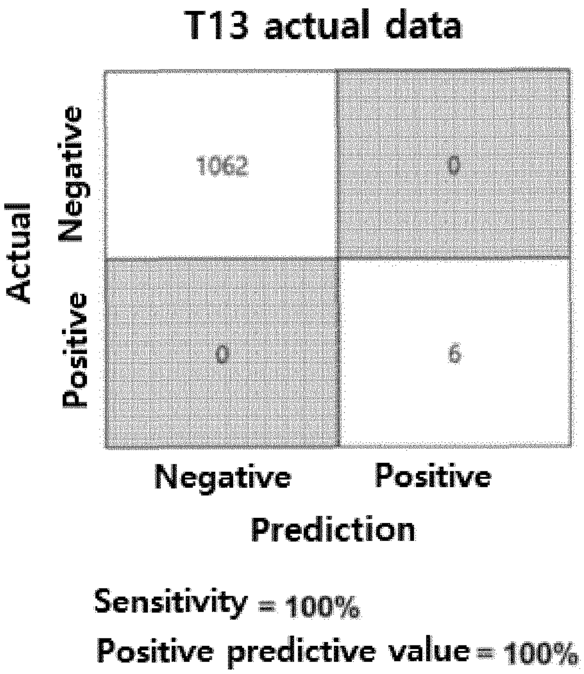

Figure 2a

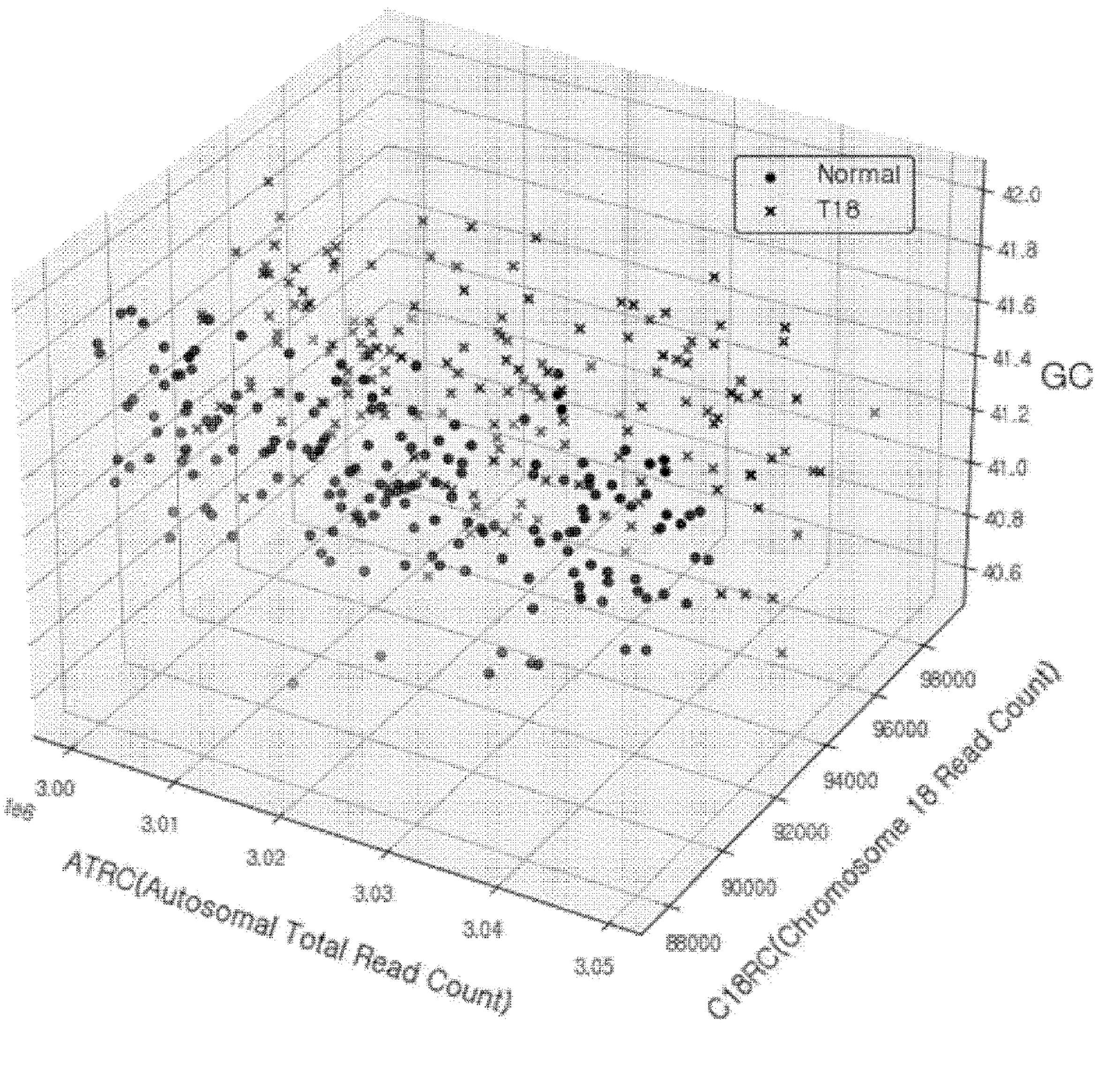
Normal
T18
42.0
41.8
41.6
41.4
41.2
41.0
40.8
40.6
GC
3.00
1e9
3.01
3.02
3.03
3.04
3.05
ATRC(Autosomal Total Read Count)
88000
90000
92000
94000
96000
98000
C18RC(Chromosome 18 Read Count)

Figure 2b

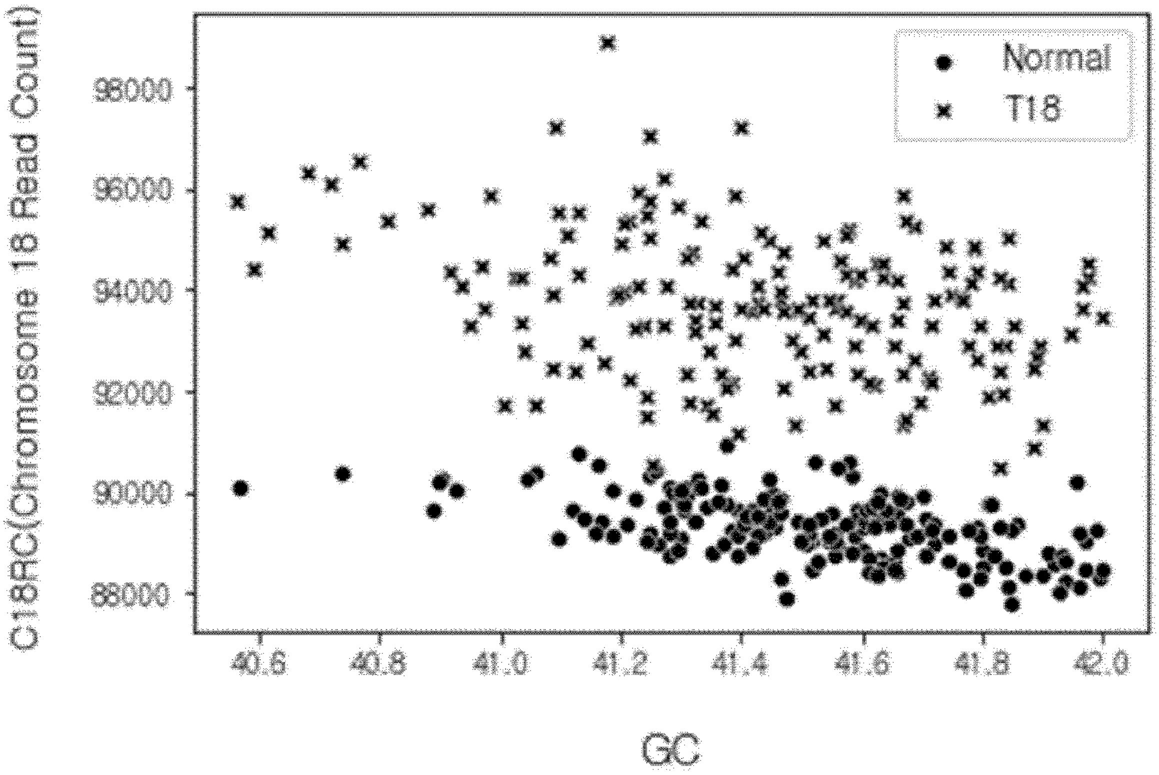
C18RC(Chromosome 18 Read Count)
98000
96000
94000
92000
90000
88000
40.6
40.8
41.0
41.2
41.4
41.6
41.8
42.0
GC
Normal
T18

Figure 2c

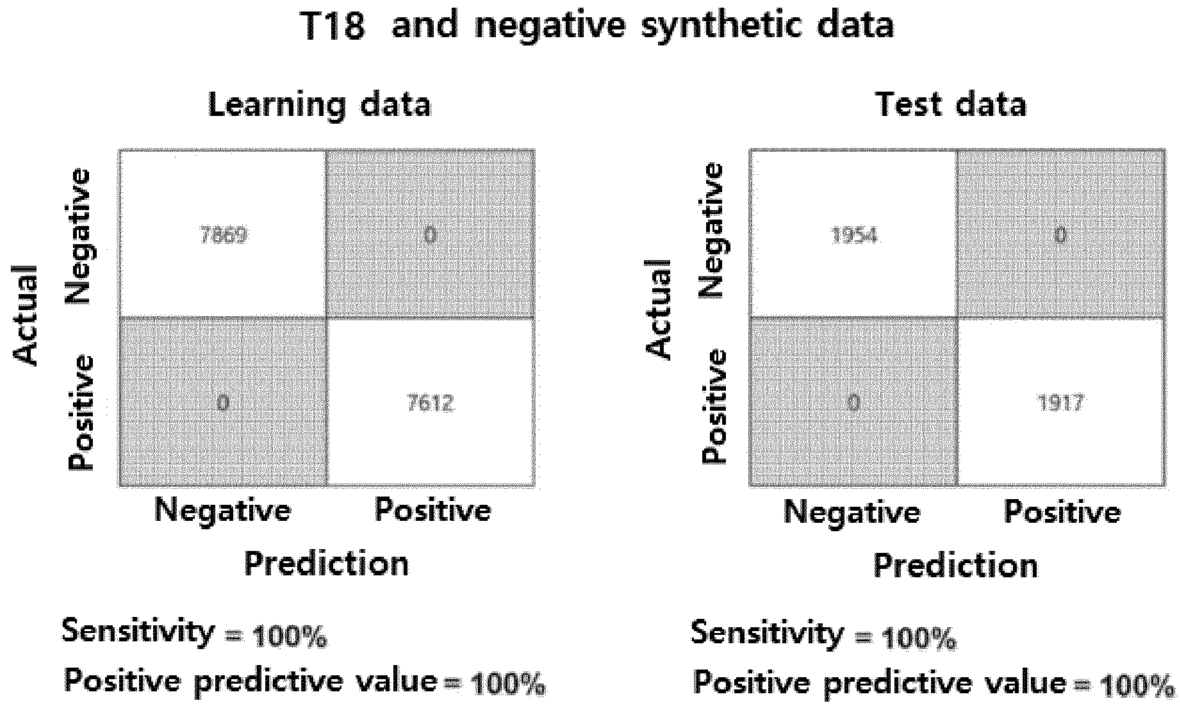


Figure 2d

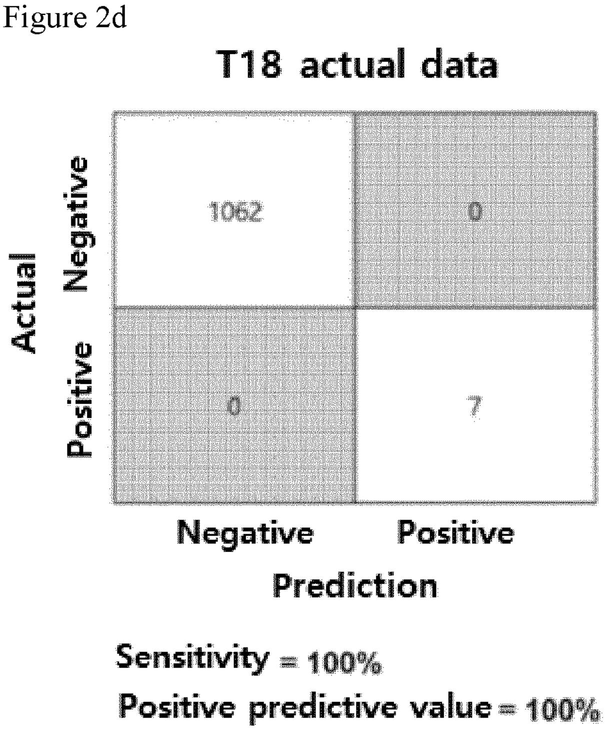

Figure 3a

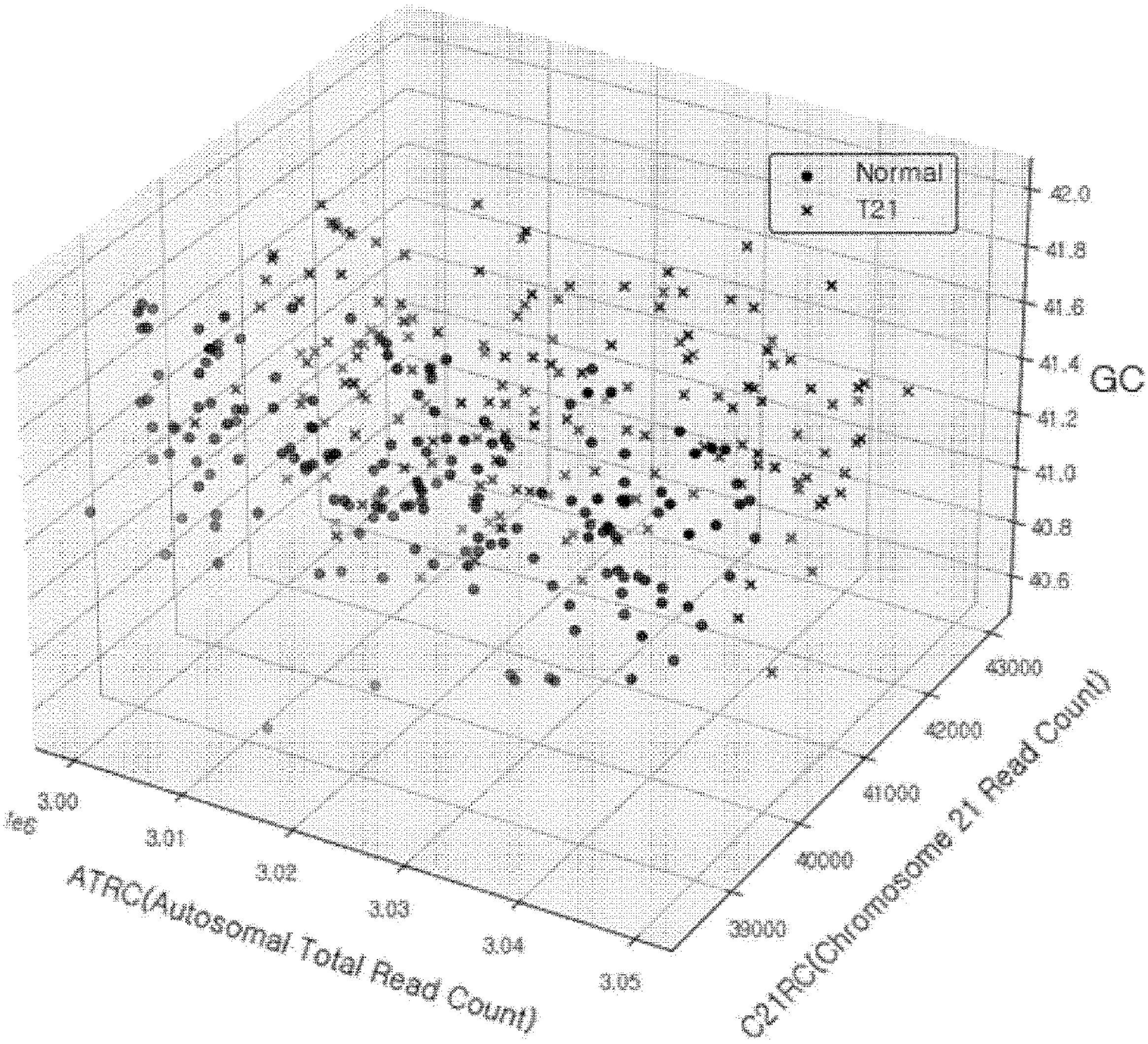
Normal
T21
42.0
41.8
41.6
41.4
41.2
41.0
40.8
40.6
GC
1e8
3.00
3.01
3.02
3.03
3.04
3.05
ATRC(Autosomal Total Read Count)
39000
40000
41000
42000
43000
C21RC(Chromosome 21 Read Count)

Figure 3b

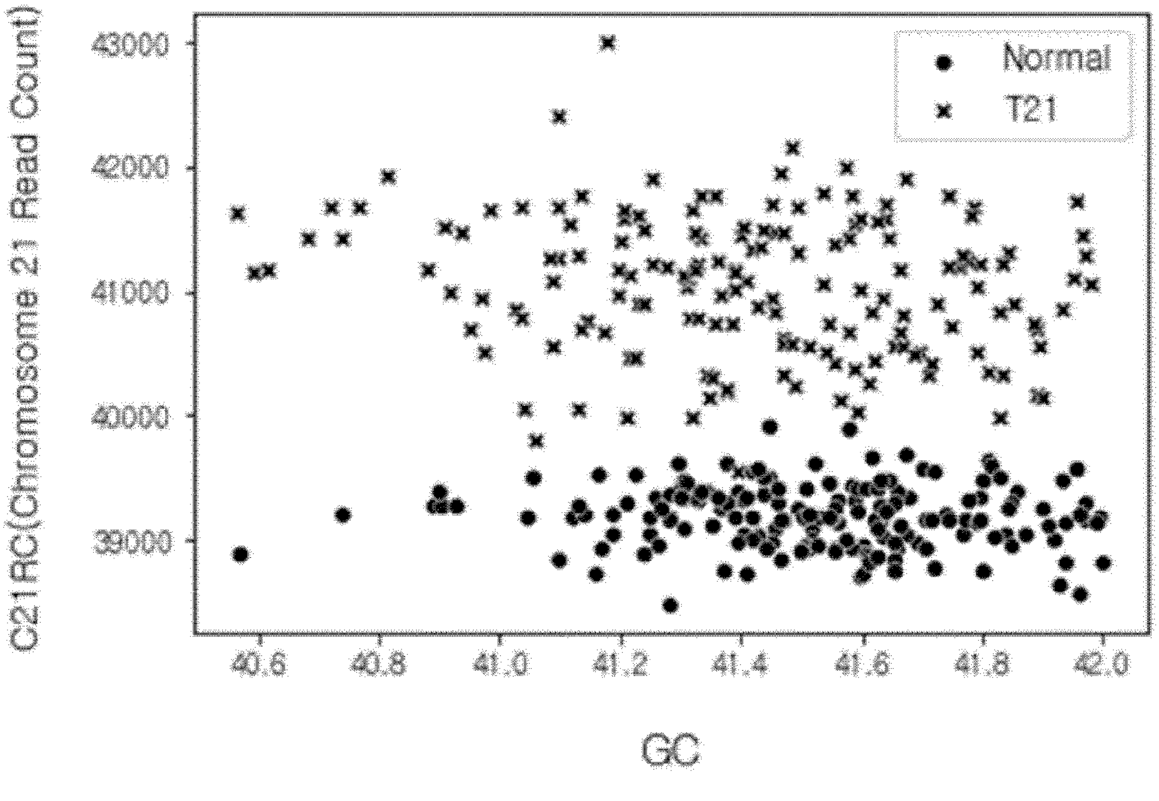
C21RC(Chromosome 21 Read Count)
43000
42000
41000
40000
39000
Normal
T21
40.6
40.8
41.0
41.2
41.4
41.6
41.8
42.0
GC

Figure 3c

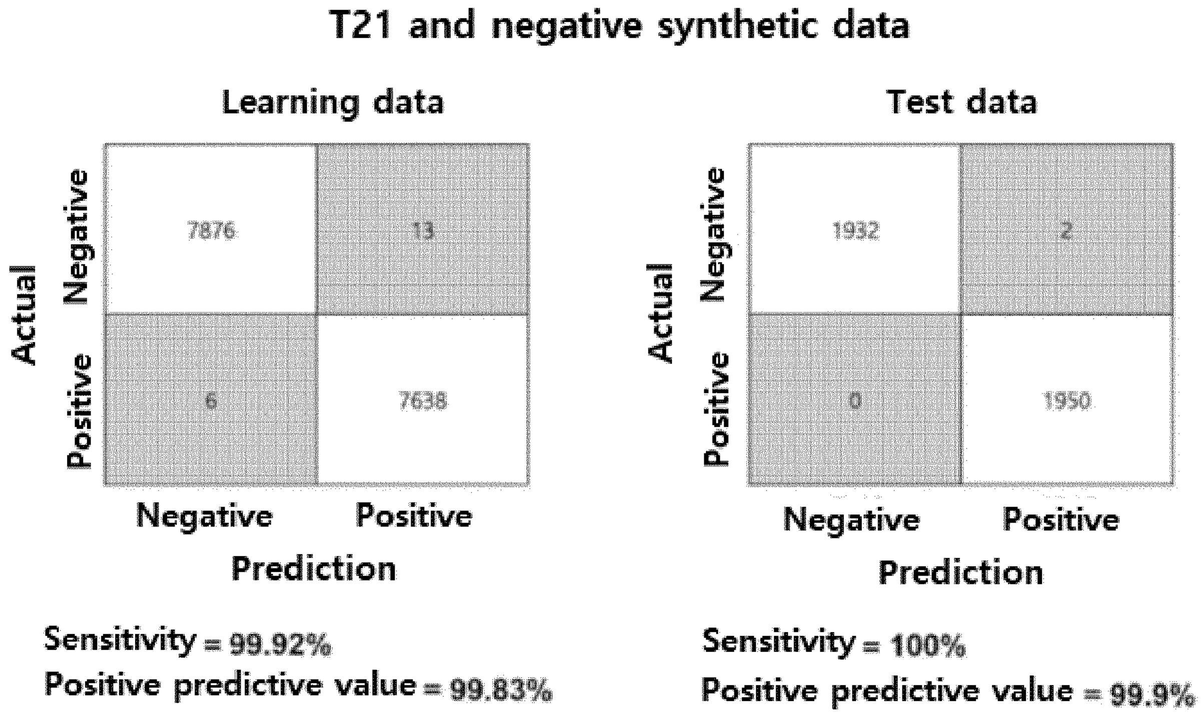


Figure 3d

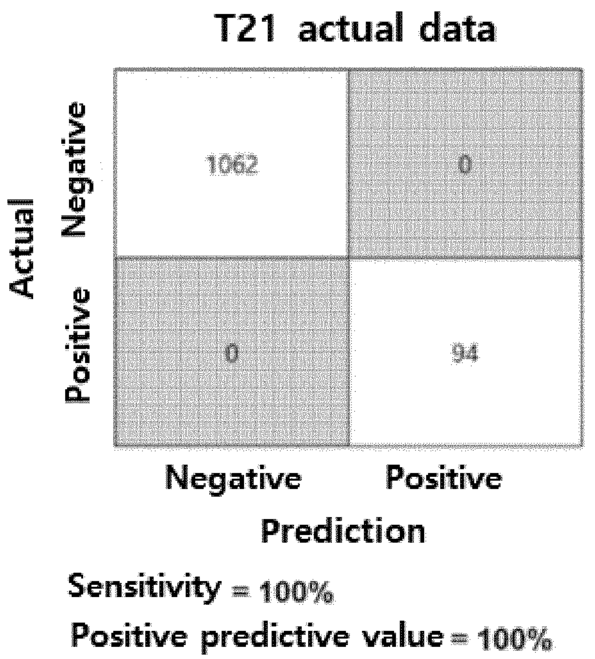

Figure 4a

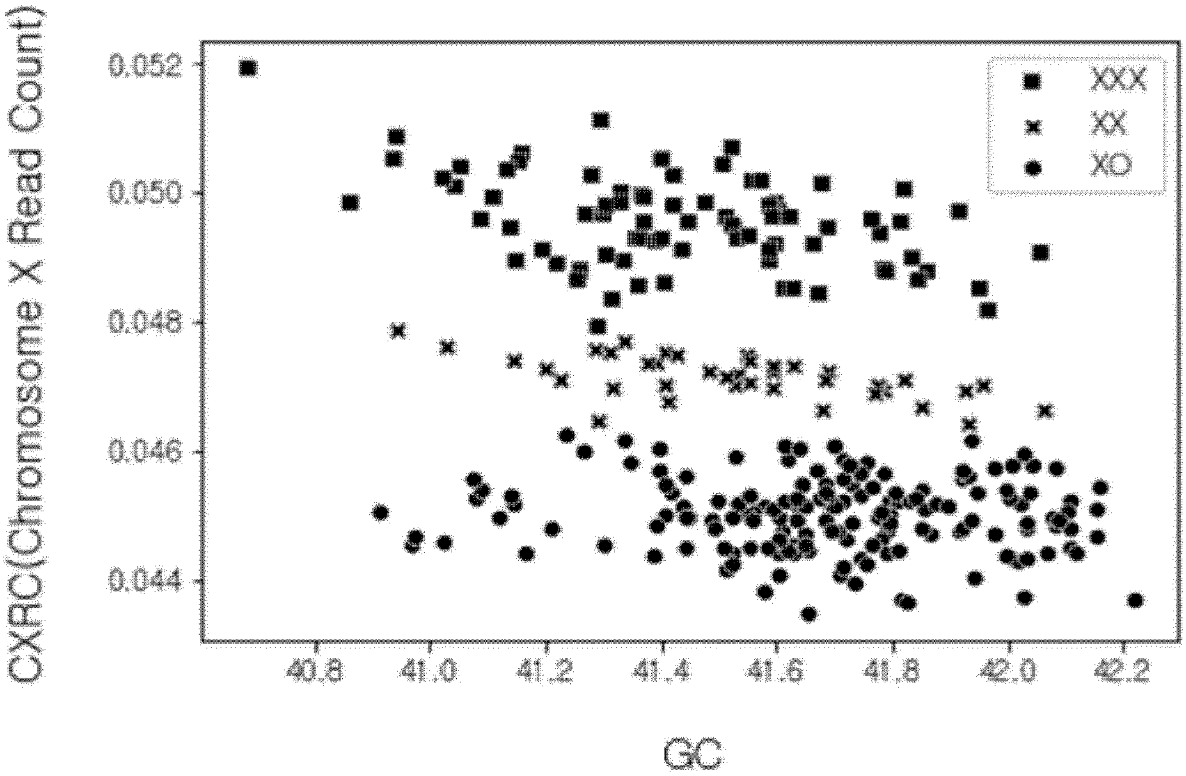


Figure 4b

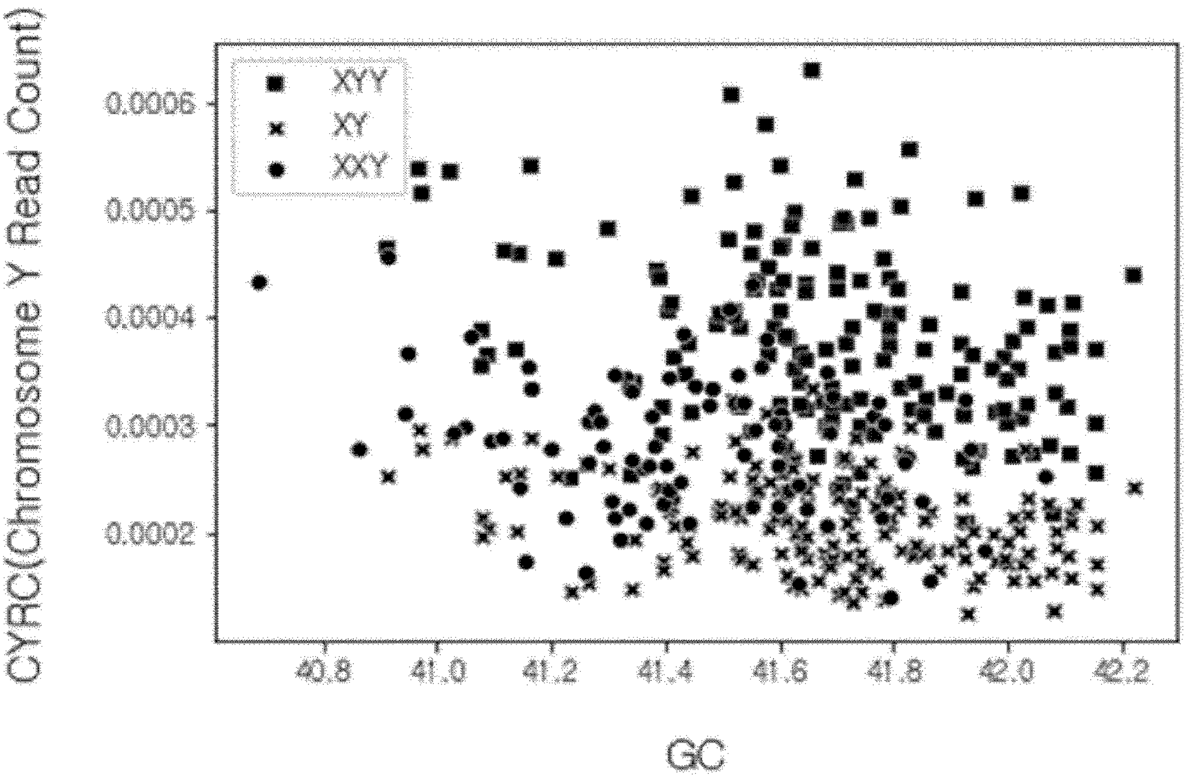

Figure 4c

## Sex chromosome positive synthetic data

### Learning data

| Actual \ Prediction | XY | XX | XO | XXY | XYY | XXX |
|---|---|---|---|---|---|---|
| XY | 7991 | 0 | 0 | 0 | 0 | 0 |
| XX | 0 | 7957 | 15 | 0 | 0 | 4 |
| XO | 0 | 5 | 8064 | 0 | 0 | 0 |
| XXY | 0 | 2 | 0 | 7953 | 7 | 0 |
| XYY | 0 | 0 | 0 | 4 | 7928 | 0 |
| XXX | 0 | 8 | 0 | 0 | 0 | 7937 |

| Item | Sensitivity (%) | Positive predictive value (%) |
|---|---|---|
| XY | 100% | 100% |
| XX | 99.76% | 99.81% |
| XO | 99.93% | 99.81% |
| XXY | 99.89% | 99.95% |
| XYY | 99.95% | 99.91% |
| XXX | 99.90% | 99.95% |
| Total | 99.90% | 99.90% |

### Test data

| Actual \ Prediction | XY | XX | XO | XXY | XYY | XXX |
|---|---|---|---|---|---|---|
| XY | 2009 | 0 | 0 | 0 | 0 | 0 |
| XX | 0 | 1951 | 3 | 0 | 0 | 0 |
| XO | 0 | 4 | 1927 | 0 | 0 | 0 |
| XXY | 2 | 0 | 0 | 1991 | 2 | 0 |
| XYY | 0 | 0 | 0 | 0 | 2068 | 0 |
| XXX | 0 | 5 | 0 | 0 | 0 | 2007 |

| Item | Sensitivity (%) | Positive predictive value (%) |
|---|---|---|
| XY | 100% | 99.90% |
| XX | 99.85% | 99.80% |
| XO | 99.79% | 99.84% |
| XXY | 99.80% | 100% |
| XYY | 100% | 99.90% |
| XXX | 99.75% | 100% |
| Total | 99.87% | 99.91% |

Figure 4d

**Sex chromosome actual data**

| Actual \ Prediction | XY | XX | XO | XXY | XYY | XXX |
|---|---|---|---|---|---|---|
| XY | 200 | 0 | 0 | 0 | 0 | 0 |
| XX | 0 | 200 | 0 | 0 | 0 | 0 |
| XO | 0 | 0 | 6 | 0 | 0 | 0 |
| XXY | 0 | 0 | 0 | 9 | 0 | 0 |
| XYY | 0 | 0 | 0 | 0 | 2 | 0 |
| XXX | 0 | 0 | 0 | 0 | 0 | 13 |

| Item | Sensitivity (%) | Positive predictive value (%) |
|---|---|---|
| XY | 100% | 100% |
| XX | 100% | 100% |
| XO | 100% | 100% |
| XXY | 100% | 100% |
| XYY | 100% | 100% |
| XXX | 100% | 100% |
| Total | 100% | 100% |

**INTERNATIONAL SEARCH REPORT**

International application No. **PCT/KR2023/004091**

**A. CLASSIFICATION OF SUBJECT MATTER**

**G16B 30/00**(2019.01)i; **G16B 20/00**(2019.01)i; **G16B 40/00**(2019.01)i; **G16B 50/00**(2019.01)i; **G16H 50/20**(2018.01)i; **G16H 50/30**(2018.01)i; **C12Q 1/6806**(2018.01)i; **C12Q 1/6827**(2018.01)i

According to International Patent Classification (IPC) or to both national classification and IPC

**B. FIELDS SEARCHED**

Minimum documentation searched (classification system followed by classification symbols)

G16B 30/00(2019.01); C12Q 1/68(2006.01); G16B 10/00(2019.01); G16B 20/00(2019.01); G16B 20/20(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 염색체이수성(chromosome aneuploidy), 리드(read), 태아 DNA 분획(fetal DNA fraction), GC 함량(GC content), 가상의 염색체이수성 데이터(virtual 六data), 기계학습(machine learning)

**C. DOCUMENTS CONSIDERED TO BE RELEVANT**

| Category* | Citation of document, with indication, where appropriate, of the relevant passages | Relevant to claim No. |
|---|---|---|
| A | KR 10-2021-0067931 A (GREEN CROSS GENOME CORPORATION) 08 June 2021 (2021-06-08) See paragraph [0096]; and claims 1 and 12. | 1-18 |
| A | WO 2017-023148 A1 (EONE DIAGNOMICS GENOME CENTER CO., LTD.) 09 February 2017 (2017-02-09) See entire document. | 1-18 |
| A | KR 10-2017-0088780 A (GENOMECARE CO., LTD.) 02 August 2017 (2017-08-02) See entire document. | 1-18 |
| A | KIM, Sunshin et al. GenomomFF: cost-effective method to measure fetal fraction by adaptive multiple regression techniques with optimally selected autosomal chromosome regions. IEEE Access. 2020, vol. 8, pp. 106880-106888. See entire document. | 1-18 |
| A | YANG, Jianfeng et al. Improving the calling of non-invasive prenatal testing on 13-/18-/21-trisomy by support vector machine discrimination. PLoS ONE. 2018, vol. 13, no. 12, e0207840, pp. 1-19. See entire document. | 1-18 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 July 2023** | **18 July 2023** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2023/004091**

| C. DOCUMENTS CONSIDERED TO BE RELEVANT | | |
|---|---|---|
| Category* | Citation of document, with indication, where appropriate, of the relevant passages | Relevant to claim No. |
| E | WO 2023-096224 A1 (THERAGEN GENOMECARE CO., LTD.) 01 June 2023 (2023-06-01) See claims 1-4, 6, 8 and 10-15. | 1-3,6-7,11,13-18 |

Form PCT/ISA/210 (second sheet) (July 2022)

**INTERNATIONAL SEARCH REPORT**

International application No.
**PCT/KR2023/004091**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☑ Claims Nos.: **19**
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2023/004091**

| Patent document cited in search report | Publication date (day/month/year) | Patent family member(s) | Publication date (day/month/year) |
|---|---|---|---|
| KR 10-2021-0067931 A | 08 June 2021 | EP 4068291 A1 | 05 October 2022 |
| | | JP 2023-504139 A | 01 February 2023 |
| | | US 2023-0028790 A1 | 26 January 2023 |
| | | WO 2021-107676 A1 | 03 June 2021 |
| WO 2017-023148 A1 | 09 February 2017 | CN 107949845 A | 20 April 2018 |
| | | KR 10-1817785 B1 | 11 January 2018 |
| | | KR 10-2017-0017384 A | 15 February 2017 |
| | | US 11339426 B2 | 24 May 2022 |
| | | US 2019-0228131 A1 | 25 July 2019 |
| KR 10-2017-0088780 A | 02 August 2017 | KR 10-1739535 B1 | 24 May 2017 |
| | | KR 10-1881098 B1 | 24 July 2018 |
| | | US 2019-0103187 A1 | 04 April 2019 |
| | | WO 2017-131359 A1 | 03 August 2017 |
| WO 2023-096224 A1 | 01 June 2023 | KR 10-2023-0076686 A | 31 May 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1739535 B
- KR 102031841


### Non-patent literature cited in the description


- **GUOLIN KE et al.** LightGBM: A Highly Efficient Gradient Boosting Decision Tree. *Proceedings of the 31st International Conference on Neural Information Processing Systems (NIPS 2017)*, 2017, 3149-3157